Europäisches Patentamt

European Patent Office

Office européen des brevets

(11) Publication number: 0 387 041
A1

# EUROPEAN PATENT APPLICATION

(21) Application number: 90302445.3

(22) Date of filing: 07.03.90

(51) Int. Cl.5: G01N 30/64, G01N 27/66

(30) Priority: 07.03.89 US 320689

(43) Date of publication of application:
12.09.90 Bulletin 90/37

(84) Designated Contracting States:
DE FR GB IT

(71) Applicant: VARIAN ASSOCIATES, INC.
611 Hansen Way
Palo Alto, CA 94303(US)

(72) Inventor: Simon, Richard Kenneth Jr.
1721 Greer Avenue
Concord, California 94521(US)

(74) Representative: Cline, Roger Ledlie et al
EDWARD EVANS & CO. Chancery House
53-64 Chancery Lane
London WC2A 1SD(GB)

(54) Nitrogen specific detector.

(57) A gas chromatography detector specific to nitrogen containing compounds is shown. The detector comprises an oxygen pyrolysis chamber for oxidizing the sample eluting from the chromatography column at high temperature and a photoionization cell for detecting the $NO_x$ species present in the oxidized sample. Due to the fact that the ionization energies of other oxides present in the sample mixture is greater than the photons created in the photoionization cell, other oxides are not detected.

FIG. I

EP 0 387 041 A1

# NITROGEN SPECIFIC DETECTOR

This invention relates to the field of gas chromatography and, in particular, to detectors used to analyze the output of gas chromatography columns after sample separation.

In gas chromatography a sample of interest is volatilized and injected into a gas chromatography column, typically housed in an oven. A carrier gas flows constantly through the column sweeping the sample along with it. Differential adsorption and desorption of the sample constituents on the partition medium in the column separates the sample into its components. Having been thus separated, the constituents of the sample elute from the end of the column at different times and flow to a detector which continuously monitors the gas flow. A change in the physical/chemical property being measured relative to the baseline property of the carrier gas signifies that a sample constituent is passing through the detector. This is commonly referred to as a "peak." A recording of the detector signal, which may contain a large number of is called a chromatogram.

A variety of detectors are available to the chromatographer for use in gas chromatography systems. The selection of what type of detector to use is a function of a variety of factors including the type(s) of samples being investigated, cost, sensitivity, selectivity and others. Some detectors respond well to a broad variety of sample species while others are useful for only specific types of compounds.

The detection of nitrogen containing compounds is frequently a goal of the chromatographer. In many instances, the chromatographer is interested in nitrogen but does not wish to detect extraneous non-nitrogen compounds. A detector which would respond only to nitrogen compounds, as opposed to a universal detector, would allow the analyst to reduce the amount of sample preparation or "clean-up" which normally would be required when handling complex samples. This is particularly useful in the analysis of nitrosamines, drugs of abuse (e.g., barbiturates, amphetamines and narcotics), pharmaceutical drugs, herbicides and pesticides, which may be present in complex sample matrices.

A variety of detectors and detection methods have been applied for nitrogen detection, including the Thermionic Specific Detector (TSD) sometimes referred to as a Nitrogen Phosphorous Detector (NPD), the Electrolytic Conductivity Detector (ECD or ELCD), the Microwave Plasma Detector or Microwave Emission Detector (MED), micro-coulometric detection and pyro-chemilluminescence nitrogen detection. Each of these detectors/detection methods have drawback when applied to nitrogen detection.

In micro-coulometric detection, the total amount of nitrogen is determined after conversion of the nitrogen compounds to ammonia via reduction on catalytic nickel and titration of the coulometrically generated hydrogen ions. The proportionality between the total number of electrons (i.e., coulombs of charge) required to maintain chemical equilibrium and the chromatographic peak area provides a means for quantifying the amount of nitrogen. This type of detector has a detection limit in the milligram to nanogram range.

The ELCD, sometimes referred to as a Hall Detector (see U.S. Pat. Nos. 4,555,383 and 4,032,296) is an improvement over the Coulson detector described in an article, *Electrolytic Conductivity Detector for Gas Chromatography*, by D.M. Coulson, (Journal of Chromatographic Science, April 1965, pp. 134-36). This type of detector operates by first oxidizing the sample in an oxygen pyrolysis furnace converting the analyte into one or more oxides. The oxides then flow to a conductivity measuring flow cell where they are dissolved in a solvent. Changes in the conductivity of the solvent are measured in the flow cell as the solution including the dissolved oxides pass by a pair of electrodes. In the Hall detector, sample conductivity is monitored at a high frequency in contrast to the d.c. mode of the Coulson detector. The ELCD has a number of problems associated with it when applied to the measurement of nitrogen. A number of interferences may be present due to the reactivity of all the oxides to the conductivity process. This requires stringent control over the pH of the solvent solution to minimize the effects of the oxides on the electrolyte. Additional scrubbers are sometimes necessary to remove undesired oxides. Since the analyte oxides are continuously being dissolved and are thereafter handled in a liquid solution, an expensive and cumbersome pumping and solvent delivery system is necessary. Typically, the solvent used is recycled continuously or otherwise presents a waste disposal problem. Recycling is accomplished by removing the interfering oxides from solution after analysis with an exchange resin. However, the optimal resin mixture is a function of the sample matrix. If not properly optimized oxides will accumulate in the solvent and interfere with the analysis. The typical solvent used in the ELCD for nitrogen analysis, i.e., n-propanol, is potentially carcinogenic. Typical detection limits for the ELCD are 5 to 10 pg N/sec.

The Thermionic Specific Detector (TSD) also known as the Nitrogen-Phosphorous Detector (NPD) operates by partially converting the analyte of interest to a reactive species, e.g., CN, which has a sufficiently high electron affinity to attach an electron from the thermionic surface of the TSD "bead". The TSD has the following problems associated with it when applied to nitrogen detection: (1) "beads" have a limited life-span; (2) it takes a long time to equilibrate and condition a new bead; (3) bead to bead response is highly variable; (4) the detector is not specific to nitrogen, but also responds to phosphorous - indeed, the quantitation of nitrogen peaks following phosphorous peaks is degraded due to excessive tailing of phosphorous compounds; (5) the detector requires frequent recalibration due to loss of bead sensitivity over time; and (6) the response of the TSD is severely hampered by the presence of halogens. The typical sensitivity of the TSD is 0.1 pg N/sec.

In a Microwave Emission Detector (MED) sample flows into a resonant microwave cavity containing a plasma generated in an inert gas, such as helium, by the application of microwave energy. The plasma fragments the sample constituents into free atoms and/or diatomic molecules which are excited to make characteristic photon emissions. Major drawbacks of the MED are its high cost, complexity, intolerance of large amounts Of hydrocarbons, and spectral interferences which occur. The typical sensitivity of the MED for nitrogen is 1 to 10 pg N/sec.

In pyro-chemilluminescence nitrogen detection (CND), which is an updated version of the thermal energy analyzer of Fine (see, *e.g.,* D.H. Fine *et al.,* "Description of the Thermal Energy Analyzer (TEA) For Trace Determination of Volatile and Non-volatile N-Nitroso Compounds", *Analytical Chemistry,* Vol. 47, p.1188, 1975), nitrogen in the analyte compounds il converted to a nitrosyl radical ($NO^-$) in an oxygen pyrolysis tube. Ozone is then mixed with the converted sample at low pressure in a relatively large (*e.g.,* 1 liter) reactor cell. Emission from the excited nitrogen dioxide occurs in the near red spectral region, and is detected by a photomultiplier tube after passing through a suitable filter (*e.g.,* a Corning CS 2-60 filter which cuts off light frequencies below 6100 Å). Problems with the CND relate to the difficulty of operating at low pressure, the need for a large reactor cell which adds considerable dead volume to the system, the difficulty of matching the spectral characteristics of the photomultiplier tube and the filter with the $NO_2$ emission, and the tendency of the excited $NO_2$ to be quenched by the other gaseous species that are present or by interaction with the walls of the reaction cell. The typical sensitivity of the CND is 100 - 500 pg N/sec.

Accordingly, it is an object of this invention to provide apparatus for measuring the presence of nitrogen in a sample stream eluting from the output of a gas chromatography column which is specific, stable, highly sensitive and relatively inexpensive.

Another object of this invention is to provide a nitrogen specific detector that does not require the use of reduced pressures, potentially carcinogenic solvents, expensive microwave equipment or monochromators.

## SUMMARY OF THE INVENTION

These and other objects are achieved by the novel design of the present invention. In its basic form, the present invention involves a method and apparatus for detecting nitrogen by first converting the nitrogen in the sample stream into NO or $NO_2$ in an oxygen pyrolyzer and then directing the pyrolyzed output to a photoionization detector (PID) which responds to the $NO_x$ but not to the other oxides in the mixture. It is important that the PID portion of the present invention be of the type which employs a lamp having a window, as opposed to a windowless PID. The presence of the window inherently limits the photons available to ion the sample to energies which are sufficient to ionize $NO_x$, but insufficient to ions other compounds produced in the oxygen pyrolyzer. As an added advantage of the present invention, the oxygen pyrolyzer may be inactivated at any time, *i.e.,* by turning off its heat source, and the detector may be operated as a standard non-selective PID. A halogen scrubber may be added between the oxygen pyrolyzer and the PID to mitigate problems associated with halogen sensitivity. Likewise, means may be added to remove water and/or carbon dioxide from the output of the oxygen pyrolyzer.

Examples of the invention will now be described with reference to the accompanying drawings in which:

FIG. 1 is a cross sectional schematic view of a oxygen pyrolyzer used in practicing the present invention.

FIG. 2 is a cross sectional schematic view of a photoionization detector used in practicing the present invention.

FIG. 3 is a chromatogram of a sample mixture analyzed using a detector of the present invention.

FIG. 4 is a chromatogram of the same sample mixture analyzed using a photoionization detector.

FIG. 5 is a chromatogram of the same sample mixture analyzed using a flame ionization detector.

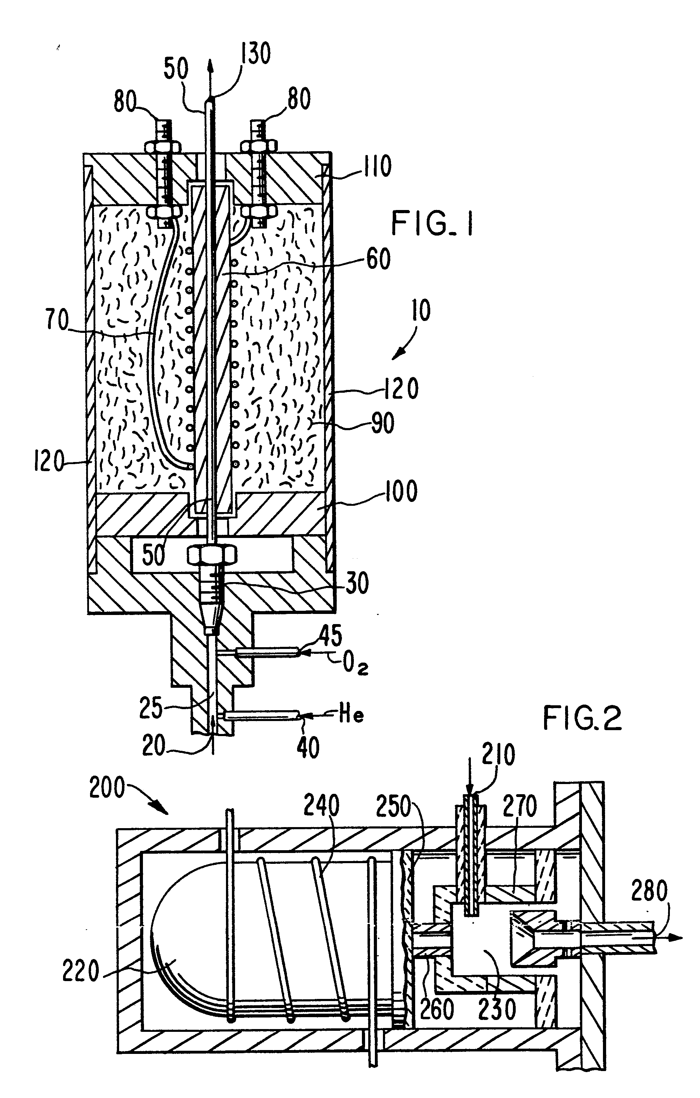

FIG. 1 shows an oxygen pyrolysis unit 10 useful in practicing the present invention. The output of a gas chromatography column is directed into cylindrical channel 25 via input 20. As is well kown by those skilled

3

in the art the dead volume between the column end and input 20 should be minimized to avoid adverse chromatographic effects. A flow of helium make-up gas is added by way of helium input 30 and oxygen is added via oxygen input 40. Typical flow rates through the oxygen pyrolysis unit are 20 - 40 ml/min. oxygen and 1 -30 ml/min. helium.

Ferrule 30 connects the sample/oxygen/helium mixture to a pyrolysis column 50. Column 50 may be made of any material capable of withstanding an oxygen environment at 800° C or above, without causing contamination of the sample. Examples of such materials include certain ceramics, quartz and inert metals. Aiumlna has been employed in a preferred embodiment because of its cost, structural capabilities, purity and availability.

Pyrolysis column 50 is surrounded by and is in good thermal contact with a thermal transfer member 60, which may also be constructed of alumina. Thermal transfer member 60 is, in turn, surrounded by ohmic heating coil 70, which is electrically connected to a voltage source via feed-through connectors 80. The heating coil 70 and thermal transfer unit 60 are surrounded by insulating material 90 and the whole unit is contained within a housing comprising a cylindrical container 120 and insulating end pieces 100 and 110. In an alternate embodiment Pyrolysis column 50 and thermal transfer member 60 may be of unitary construction.

In a preferred embodiment, the ohmic heating coil is designed to produce a temperature of at least 800° C within pyrolysis tube 50. At this temperature the analyte species undergo simple oxidation in the presence of oxygen. In particular, any nitrogen in the sample is converted to NO or $NO_2$. After oxidation within the pyrolysis tube 50, the sample flows to outlet 130. Pyrolysis column 50 may be packed with a platinum mesh (not shown) to increase the conversion efficiency of the column.

FIG. 2 shows a photoionization detector (PID) 200. The output of oxygen pyrolysis unit 10 flows via outlet 130 to PID inlet 210 via connecting tubing not shown. Again, efforts should be made to minimize the dead volume between outlet 130 and inlet 210. PID 200 comprises two major elements - photon source 220 and photoionization cell 230.

Photon source 220 is a bulb of the type that is well known in the art. The bulb is typically filled with helium, xenon or krypton at reduced pressure (e.g., 1 - 5 torr) which is excited to create a plasma by rf current in induction coil 240. When excited gas atoms relax to a ground state they emit high energy photons in the UV portion of the spectrum. The energy of the photons which can escape from the bulb is limited a maximum of about 10.6 eV by the presence of a window 250. Photons which escape through the window travel through tube 260 and into the photoionization cell 230. Tube 260 also serves as the polarizing or biasing electrode for the PID via an electrical connection (not shown). The photons entering the photoionization cell 230 strike the sample species which have entered the cell via inlet 210, selectively ionizing those species which have an ionization potential of 10.6 eV or less. A collecting electrode 270 connected to an electrometer measures changes in current in the cell corresponding to the presence of ionized species. Methods for measuring and recording changes in the ion current in the cell 230 are well known to those skilled in the art and need not be described in detail.

After transiting the photoionization cell, the sample gas is exhausted via outlet 280.

In alternate embodiments, additional "sweep" gas may be added to the photoionization cell (not shown) to minimize band broadening within the cell. Another alternative would be to use a swept gas window, as is know in the art, to operate as a diffusion barrier. Likewise, it is well known to substitute a capacitively coupled or even a direct current lamp in lieu of the inductively coupled lamp as shown.

Windowless photoionization detectors of the type that are known in the prior art, (see, e.g., US. Pat. No. 4,266,196) are not useful in practicing the present invention because they emit photons having too high an energy. The reasons for this will become clear after considering the following.

Of the commonly chromatographed compounds, only NO and $NO_2$ have an ionization potential below 10.6 eV after undergoing oxygen pyrolysis. This is shown in the following Table 1:

TABLE 1

| Ionization Potential of Products of Oxygen Pyrolysis | | |
|---|---|---|
| COMPOUND | FORMULA | IONIZATION POTENTIAL |
| Carbon Monoxide | $CO$ | 14.01 eV |
| Carbon Dioxide | $CO_2$ | 13.79 eV |
| Carbon Oxysulfide | $COS$ | 11.17 eV |
| Chlorine Monoxide | $ClO$ | 10.40°eV |
| Chlorine Dioxide | $ClO_2$ | 11.10 eV |
| Cyano | $CN$ | 14.60 eV |
| Cyanogen | $C_2N_2$ | 13.60 eV |
| Hydrogen Bromide | $HBr$ | 11.62 eV |
| Hydrogen Chloride | $HCl$ | 12.90 eV |
| Hydrogen Peroxide | $H_2O_2$ | 11.26 eV |
| Hydroperoxy | $HO_2$ | 11.53 eV |
| Hydroxyl | $OH$ | 13.53 eV |
| Meta-Phosphoric Acid | $HPO_3$ | 11.20 eV |
| Nitric Oxide | $NO$ | 9.25°eV |
| Nitrogen Dioxide | $NO_2$ | 9.78°eV |
| Nitrous Oxide | $N_2O$ | 12.90 eV |
| Oxygen | $O_2$ | 12.08 eV |
| Oxygen Fluoride | $OF$ | 13.00 eV |
| Ozone | $O_3$ | 12.80 eV |
| Phosphorous Monoxide | $PO$ | 16.30 eV |
| Phosphorous Trioxide | $PO_3$ | 13.50 eV |
| Sulfur Monoxide | $SO$ | 12.10 eV |
| Sulfur Dioxide | $SO_2$ | 12.34 eV |

From the foregoing Table I it is evident that of the common products likely to be present in the output of a oxygen pyrolysis unit of the type shown in FIG. 1, only those marked with an asterisk (*), i.e., chlorine monoxide, nitric oxide, and nitrogen dioxide, will be ionized by a 10.6 eV PID lamp. If the possible chlorine interference is of concern it is possible to either scrub the pyrolyzer output to remove halogen compounds or to use a 9.6 eV lamp. 9.6 eV lamps are commonly available for use in PIDs. However, use of such a lamp will reduce thee nitrogen sensitivity because it is below the photoionization potential of one of the pyrolysis products, nitrogen dioxide.

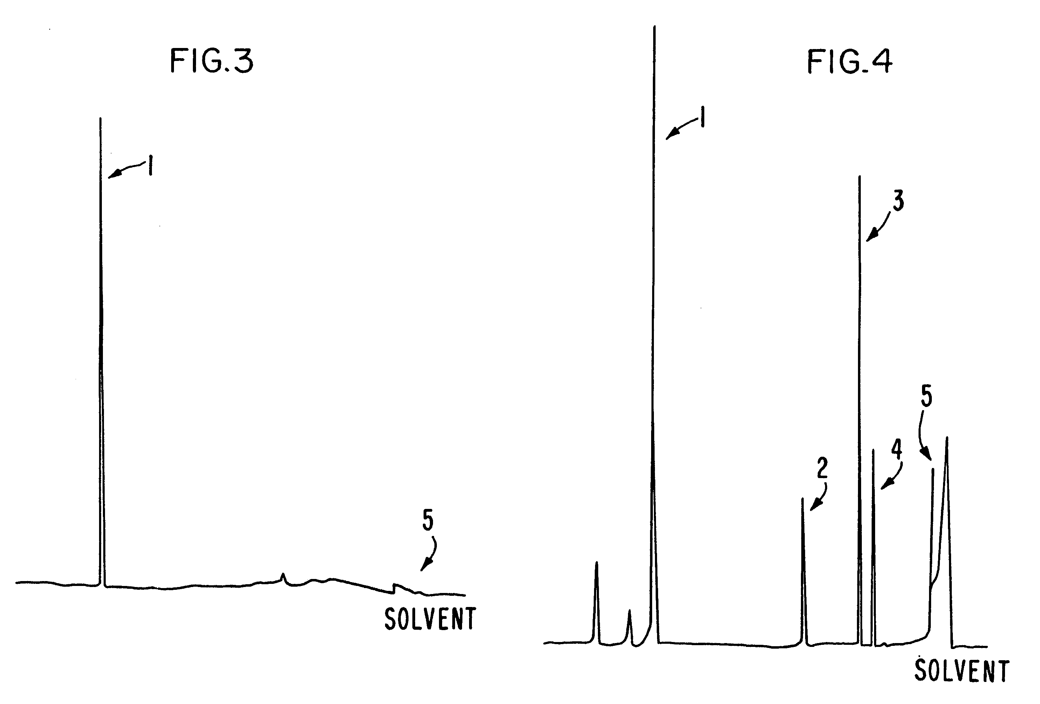

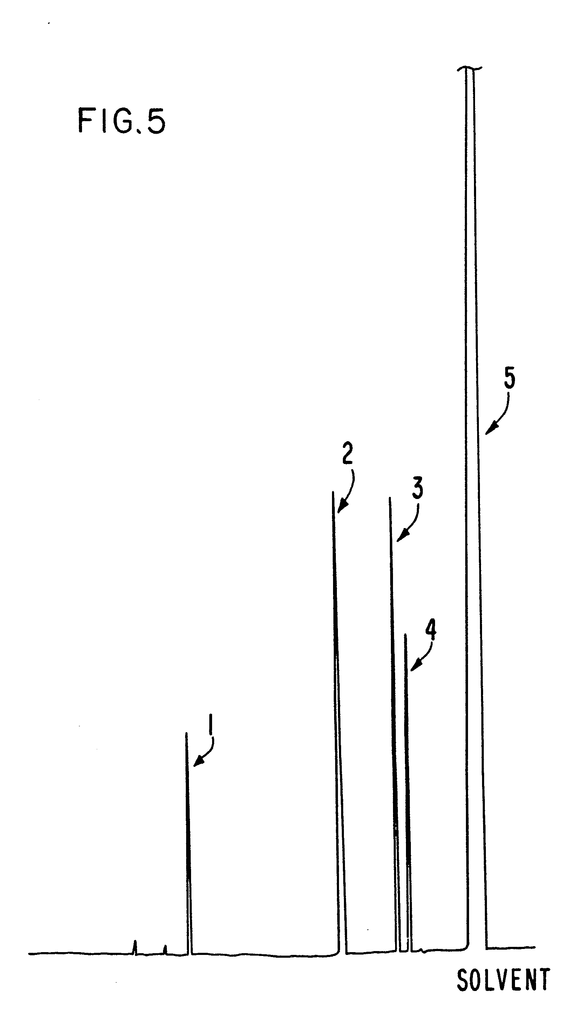

FIGS. 3, 4 and 5 are three chromatograms of a 1 μL injection of identical sample mixture analyzed using the same chromatography column but with three different detectors. FIG. 3 was produced using a detector of the present invention. FIG. 4 was produced with a photoionization detector (PID), and, in particular the PID portion of the present invention (i.e., with the oxygen pyrolyzer off). FIG. 5 was done for reference using a flame ionization detector which is a standard universal detector. In each instance peak 1 is the nitrogen containing compound azobenzene. Note that this is the only significant peak which appears in the FIG. 3 chromatogram. A slight solvent peak 5 (iso-octane) is barely noticeable in FIG. 3. Other major peaks in FIGS. 4 and 5 include peak 2 (triethylphosphate), peak 3 (thiolbenzene), and peak 4 (thiolhexane). It is calculated that these chromatograms show selectivity of the present invention for nitrogen versus carbon, phosphorus and sulfur in excess of 10,000:1. Sensitivity is estimated at 60 pg N/mL, and it is believed that this can be improved by optimizing certain parameters such as flow rates and lamp illuminance.

Since many changes could be made in the above construction and many apparently widely different modifications and embodiments of this invention can be made without departing from the scope thereof, it is intended that all matter contained in the above description or shown in the accompanying drawings shall be interpreted as illustrative and not in a limiting sense.

**Claims**

5

1. A detector for use in a gas chromatography system comprising:

oxygen pyrolysis means for continuously heating in the presence of oxygen a sample stream eluting from the output of a gas chromatography column, whereby the sample stream undergoes oxygen pyrolysis,

photoionization means, downstream of said oxygen pyrolysis means, for bombarding said sample stream with photons having energies no greater than a selected threshold value,

current measuring means for detecting changes in the ion density of the sample stream subject to photon bombardment.

2. The detector of claim 1 wherein said photon energy threshold value is selected so that substantially the only compounds in said sample stream which photoionize are nitrogen dioxide and nitric oxide.

3. The detector of claim 1 wherein said photoionization means comprises a lamp having a maximum photon energy output of 10.6 eV.

4. The detector of claim 1 wherein said photoionization means comprises a lamp having a maximum photon energy output of 9.6 eV.

5. The detector of claim 1 wherein said oxygen pyrolysis means comprises an inert tube having inlet and outlet ends surrounded by heating means and connected at its inlet end to a source of oxygen and to the output of said gas chromatography column and connected at its outlet end to the input of said photoionization means.

6. The detector of claim 4 wherein said heating means comprises an ohmic heating coil capable of heating the interior of said tube to a temperature of at least $800^\circ$ C.

7. A nitrogen detector comprising:

an oxygen pyrolysis cell for converting at least part of any nitrogen which may be present in a sample stream into $NO_x$, and

a photoionization detector of the type employing a lamp as a photon source, wherein the maximum energy of emitted photons is no greater than approximately 10.6 eV,

whereby substantially the only compounds in the sample which are ionized by said photon source is said $NO_x$.

8. The nitrogen detector of claim 7 wherein said oxygen pyrolysis cell comprises a pyrolysis tube having inlet and outlet ends, said inlet end being connected to a sample source and an oxygen source and said outlet end being connected to the input of said photoionization detector.

9. The nitrogen detector of claim 8 wherein said pyrolysis tube is surrounded by heating means capable of maintaining the interior of the pyrolysis tube at a temperature of at least $800^\circ$ C.

10. A method of detecting the presence of nitrogen in a sample stream eluting from the end of a gas chromatography column comprising the steps of, continuously heating the flowing sample stream in the presence of oxygen so that at least some of any nitrogen in the sample stream is converted to $NO_x$ and thereafter, flowing the oxidized sample to a photoionization detector of the type employing a lamp photon source,

whereby substantially only the nitrogen compounds in the sample stream produce a response in the photoionization detector.

11. The nitrogen detector of Claim 9 wherein said heating means comprises an ohmic heating coil in thermal contact with said pyrolysis tube.

FIG_1

FIG.2

# FIG.3

# FIG.4

EP 0 387 041 A1

FIG.5

SOLVENT

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| Y | US-A-4 295 856 (R.J. ANDERSON)<br>* abstract; figure 2 *<br>--- | 1-11 | G 01 N 30/64<br>G 01 N 27/66 |
| Y | AU-A-4 035 972 (WALDEN RESEARCH CORPORATION)<br>* whole document *<br>--- | 1-11 | |
| A | INTERNATIONAL LABORATORY<br>vol. 17, no. 7, September 1987, pages 68-77, Shelton, US; J.N. DRISCOLL: "A photoionization detector optimized for capillary column GC" * figure 1 *<br>--- | 1,7 | |
| A | US-A-3 933 432 (J.N. DRISCOLL)<br>* whole document *<br>--- | 1,7 | |
| A | M. OEHME: "GAS-CHROMATOGRAPHISCHE DETEKTOREN"<br>1982, pages 66-73,88,89, Hüthig, Heidelberg, D * pages 67,68,71,72,88,89 *<br>----- | 1,7 | |

**TECHNICAL FIELDS SEARCHED (Int. Cl.5)**

G 01 N

The present search report has been drawn up for all claims

| Place of search | Date of completion of the search | Examiner |
|---|---|---|
| BERLIN | 12-06-1990 | BRISON O.P. |